# FASCICULE DE BREVET EUROPEEN

(11) **EP 1 273 272 B1**
(45) Date de publication et mention de la délivrance du brevet: **19.10.2005**
(21) Numéro de dépôt: 02291639.9
(22) Date de dépôt: 01.07.2002
(51) Int. Cl.: A61B 17/86, F16B 31/02, B25B 23/14

(54) **Dispositif de solidarisation de parties osseuses**
Befestigungsvorrichtung für Knochenteile
Fixing device for bone portions

(30) Priorité: 02.07.2001 US 301858 P
(43) Date de publication de la demande: 08.01.2003
(73) Titulaire: Depuy France, 69800 Saint Priest (FR)
(72) Inventeur: Weil, Lowell Scott Sr., Des Plaines, IL 60016-1250 (US); Godest, Anne Céline, 69008 Lyon (FR)
(74) Mandataire: Bernasconi, Jean Raymond

(56) Documents cités:
- EP-A- 0 857 465
- FR-A- 2 765 795
- FR-A- 2 788 960
- FR-A- 2 808 182

## Description

La présente invention a trait à un dispositif de solidarisation de parties osseuses, telles que, notamment, des parties de tête de métatarsien, par exemple, dans le cadre de la reprise par l'ostéotomie de Weil.

L'ostéotomie de Weil est l'opération consistant à effectuer, sur une tête métatarsienne, un trait parallèle à la plante du pied et à décaler une partie vers l'arrière, puis à solidariser les parties osseuses.

On utilise couramment, pour réunir des fragments ou éléments osseux de petite taille, des vis chirurgicales contenant des tronçons filetés destinés à se visser dans les deux parties osseuses destinées à être maintenues l'une par rapport à l'autre, et il est connu de donner aux filetages présents sur de telles vis, des pas différents afin d'assurer un bon ancrage et un effet de compression favorable à la reprise osseuse et à l'ostéo-intégration. De telles vis sont décrites, par exemple, dans le brevet français FR 2 788 960 sur lequel est basé le préambule de la revendication 1.

Ces vis réalisées, par exemple en un matériau bio-compatible, tel que le titane, sont difficiles à manier de façon précise, notamment en raison de leur petite taille. Elles présentent souvent une construction compliquée qui en augmente le prix de revient. Enfin, elles nécessitent l'utilisation d'ancillaires, généralement encombrants et mal adaptés à une vérification précise de la position exacte de la vis mise en place dans les fragments osseux.

La présente invention se propose de remédier à ces inconvénients et de fournir un dispositif de solidarisation osseuse particulièrement facile à manier et à poser et assurant une excellente consolidation.

L'invention a pour objet un dispositif de solidarisation de parties osseuses entre elles, notamment de parties osseuses de petite taille, par exemple des parties de tête de métatarsiens, qui se présente sous la forme d'une broche comprenant une tige allongée, suivie, après une zone d'affaiblissement de matière permettant la sécabilité, d'un élément de fixation s'étendant de façon tronconique pour diminuer de diamètre en direction de l'extrémité antérieure libre de l'élément, et présentant, sur cette surface tronconique, un filetage de pas progressivement décroissant à partir de ladite extrémité antérieure et dont les sommets de filets, dont les fonds sont formés par ladite surface tronconique, sont inscrits dans un cylindre géométrique co-axial à l'axe de la broche, ladite extrémité antérieure étant biseautée de façon à former une extrémité auto-forante, afin de permettre un vissage auto-taraudant dans les parties osseuses.

Il en résulte une augmentation croissante de profondeur de filet depuis la zone d'affaiblissement vers l'extrémité antérieure libre.

La partie postérieure de l'élément de fixation, qui s'étend vers la zone d'affaiblissement, présente des rainures longitudinales interrompant partiellement les filets dans cette zone, afin de permettre l'introduction de doigts correspondant d'un outil permettant, s'il y a lieu, le dévissage de l'élément de fixation par rotation en sens inverse de la rotation qui a permis son enfoncement dans les parties osseuses.

L'extrémité auto-forante est, de préférence, réalisée par une pointe de type trocard, formée, par exemple, de trois pans inclinés et décalés angulairement de 120°.

De préférence, la tige de la broche, qui est reliée à l'élément de fixation propre dit par un affaiblissement déterminant une zone sécable, présente une surface cylindre lisse ou autrement traitée pour faciliter la prise dans un appareil rotatif tel qu'une perceuse.

La partie sécable est avantageusement réalisée sous forme d'une gorge à section sensiblement triangulaire dont la pointe détermine un fond de gorge sensiblement aigu, qui facilite la rupture, par basculement angulaire, de l'élément de fixation par rapport à la tige, tout en assurant une parfaite résistance aux sollicitations générées lors de l'enfoncement de l'élément de fixation dans les parties osseuses.

La broche selon l'invention peut ainsi être utilisée pour réunir et appliquer l'un contre l'autre deux fragments osseux après positionnement respectif de ceux-ci par le chirurgien, puis forage préalable éventuel d'un trou avec une broche de diamètre plus petit et introduction et vissage compressif de la broche selon l'invention jusqu'à sa position finale, suivie d'une rupture de la zone sécable, et de l'éloignement de la tige proprement dite de la broche.

Dans un mode de mise en oeuvre avantageux, on peut, pour forer le trou préalable, utiliser, une broche filetée de diamètre inférieur, après quoi l'on met en place la broche selon l'invention, ce qui provoque, entre les filets, des déplacements de petits volumes de matières osseuses facilitant l'ostéo-intégration.

De préférence, et conformément à l'invention, on regroupe, dans un jeu, une pluralité de broches selon l'invention ayant des dimensions différentes en longueur et/ou en diamètre de sommet de filet et/ou en conicité et/ou en pas.

De préférence, les broches d'un jeu se distinguent par la longueur de l'élément de fixation implantable, le diamètre de sommet de filetage restant inchangé.

Le nombre de différentes tailles de broches d'un tel jeu ou kit est avantageusement compris entre 10 et 16, le jeu pouvant avoir, pour chaque taille, une ou plusieurs broches.

De préférence les dimensions du dispositif selon l'invention s'étendent dans les plages suivantes :
- longueur de l'élément de fixation, entre sa pointe et le fond de gorge sécable : 9mm à 40mm.
- conicité de l'élément de fixation 0,75° ± 0,5°
- diamètre moyen de la surface tronconique : 1,2mm
- diamètre du cylindre géométrique de sommet de filet : 2mm à 2,5mm
- variation de pas de filet (de) 0,5mm à la pointe à 1,8mm à l'extrémité postérieure, ces valeurs pouvant varier de plus ou moins 0,5mm.

De préférence les sections de filets sont sensiblement triangulaires avec une face inclinée vers l'avant de l'élément de fixation et une face sensiblement perpendiculaire à l'axe de la broche vers l'arrière de l'élément de fixation.

D'autres avantages et caractéristiques de l'invention apparaîtront à la lecture de la description suivante, faite à titre d'exemple non limitatif et se référant au dessin annexé dans lequel :
la figure 1 représente une vue en élévation d'une broche de faible longueur selon l'invention,
la figure 2 représente une vue agrandie de l'extrémité antérieure de la broche,
la figure 3 représente une vue en coupe III-III et agrandie, de la figure 2,
la figure 4 représente une vue agrandie, en élévation, de la partie comprenant la zone de rupture,
la figure 5 représente une vue similaire à la figure 1 pour broche de grande longueur,
la figure 6 représente une vue analogue à la figure 2 pour cette broche,

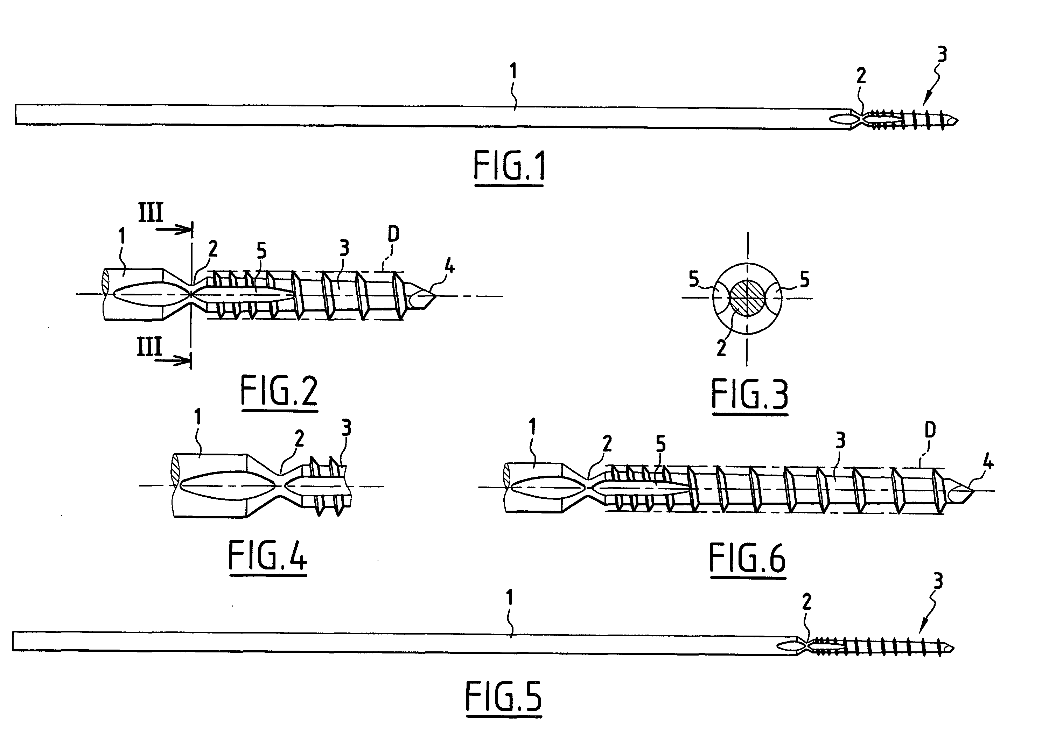

On se réfère tout d'abord aux figures 1 à 4.

On a représenté sur ces figures une forme de réalisation d'une broche selon l'invention réalisée en titane de nuance TA6V, de norme ISO 5832/3 et ayant une longueur de 114 mm. Cette broche comporte une longue tige cylindrique 1 aboutissant à une gorge triangulaire 2 formant une zone d'affaiblissement sécable. Depuis le fond de cette gorge triangulaire 2 s'étend l'élément de fixation proprement dit 3.

Cet élément possède, comme on le voit mieux sur la figure 2, une forme tronconique, ayant une conicité faible, et se termine à l'extrémité antérieure par une pointe en trocard 4 formée de trois pans biseautés et décalés angulairement de 120° autour de l'axe longitudinal de la broche.

Depuis l'extrémité antérieure, et plus précisément depuis l'arrière de la pointe 4, la broche comporte un filetage dont les flancs antérieurs de filet s'inclinent vers l'avant et vers l'intérieur, alors que les flancs postérieurs sont sensiblement perpendiculaires à l'axe. Comme on le voit sur la figure 2, le pas du filetage diminue progressivement depuis la valeur 1,76 mm jusqu'à la valeur de 0,50 mm à l'arrière pour le dernier tour de filet.

Le diamètre de fond de gorge à l'extrémité antérieure est de 1 mm et de 1,4 mm à l'extrémité postérieure, le diamètre moyen étant ainsi de 1,2 mm dans la zone centrale de l'élément 3. Conformément à l'invention les sommets de filets sont tous inscrits dans un cylindre géométrique D ayant un diamètre de 2 mm.

Ainsi la profondeur de la gorge du filetage croît progressivement vers l'avant, formant des filets de hauteur croissante pour assurer un ancrage fortement amélioré, qui, combiné au serrage progressif assuré par la variation du pas du filetage, augmente considérablement la cohésion, puis l'ostéointégration des fragments osseux rassemblés.

Au niveau de la gorge 2, on a fraisé deux rainures longitudinales, diamétralement opposées, 5, dont la profondeur n'excède pas la profondeur de la gorge 2, comme on le voit notamment sur la figure 3. Ces rainures s'étendent, en partie, dans l'extrémité antérieure de la tige 1 proprement dite et s'étendent sur une grande partie de la moitié postérieure de l'élément 3.

La mise en oeuvre de la broche selon l'invention s'effectue de la façon suivante :

Pour l'ostéotomie de Weil, le chirurgien rapproche les deux fragments osseux de métatarsien à réunir et les fixe provisoirement à l'aide de daviers dans la position qu'ils doivent occuper. Si nécessaire, il fore ensuite, à l'emplacement prévu pour recevoir l'élément 3, un trou avec un forêt ou une broche de préférence filetée et dont le diamètre extérieur est inférieur au diamètre de l'élément 3. Il mesure ensuite, avec une réglette, la longueur de l'élément 3 souhaité. Après avoir extrait la broche de forage, il place la broche 1 de dimension appropriée dans le mandrin d'un appareil tel qu'une perceuse et visse l'élément 3 formant l'extrémité de la broche dans le trou préalablement foré. Lors de l'enfoncement de l'élément 3, les fragments osseux à réunir se trouvent progressivement pressés l'un vers l'autre. Le chirurgien vérifie ensuite que l'élément 3 est enfoncé de la quantité désirée, en vérifiant la position du fond de gorge 3 par rapport à la corticale osseuse.

Après cela, il plie la tige 1 par rapport à l'élément 3, ce qui provoque une rupture nette dans la zone 2 et l'élément 3 reste ainsi dans sa position définitive.

Si le chirurgien souhaite extraire l'élément 3 il utilise une fraise-cloche pour dégager le pourtour de l'extrémité postérieure de l'élément 3, puis utilise un ancillaire en forme de tournevis présentant en son extrémité antérieure deux doigts parallèles en direction longitudinale, susceptibles de venir se placer dans les deux rainures longitudinales 5 de l'extrémité postérieure de l'élément 3 où ils prennent appui. Le dévissage s'effectue alors facilement, la rotation provoquant l'extraction de l'élément de fixation 3.

En se référant à la figure 4 on a présenté, en utilisant les mêmes chiffres de référence, une broche ayant une partie 3 de grande longueur, les dimensions en diamètre restant identiques. La rainure longitudinale 5 présente, de préférence, une longueur identique à celle de la rainure longitudinale 5 de la figure 2, de sorte qu'elle n'occupe plus la plus grande partie de la moitié postérieure de l'élément 3.

## Revendications

1. Dispositif de solidarisation de parties osseuses entre elles, notamment de partie osseuses de petite taille, par exemple des parties de tête de métatarsiens, qui se présente sous la forme d'une broche comprenant une tige allongée (1), suivie, après une zone d'affaiblissement de matière (2) permettant la sécabilité, d'un élément de fixation (3) s'étendant de façon tronconique pour diminuer de diamètre en direction de l'extrémité antérieure libre (4) de l'élément, et présentant, sur cette surface tronconique, un filetage de pas progressivement décroissant à partir de ladite extrémité antérieure et dont les sommets de filets, dont les fonds sont formés par ladite surface tronconique, sont inscrits dans un cylindre géométrique (D) co-axial à l'axe de la broche, ladite extrémité antérieure (4), étant biseautée de façon à former une extrémité forante, afin de permettre un vissage dans les parties osseuses,
**caractérisé en ce que** la partie postérieure de l'élément de fixation (3), qui s'étend vers la zone d'affaiblissement (2), présente des rainures longitudinales (5) interrompant partiellement les filets dans cette zone, afin de permettre l'introduction de doigts correspondant d'un outil permettant le dévissage de l'élément de fixation par rotation en sens inverse de la rotation auto-taraudante.

2. Dispositif selon la revendication 1, **caractérisé en ce que** la partie sécable est réalisée sous forme d'une gorge (2) à section (4) sensiblement triangulaire dont la pointe détermine un fond de gorge sensiblement aigu, qui facilite la rupture, par basculement angulaire, de l'élément de fixation par rapport à la tige, tout en assurant une parfaite résistance aux sollicitations générées lors de l'enfoncement de l'élément de fixation dans les parties osseuses.

3. Dispositif selon l'une des revendications 1 à 2 **caractérisé en ce que** les sections de filets sont sensiblement triangulaires avec une face inclinée vers l'extrémité antérieure (4) de l'élément de fixation et une face sensiblement perpendiculaire à l'axe de la broche vers la partie postérieure de l'élément de fixation (3).

4. Jeu de dispositifs selon l'une des revendications 1 à 3, **caractérisé en ce qu'**il comporte une pluralité de dispositifs dont les dimensions de l'élément de fixation (3) varient selon l'un, au moins, des paramètres : longueur, diamètre de sommet de filet, conicité, pas.

5. Jeu selon la revendication 4, **caractérisé en ce que** les dispositifs du jeu présentent des longueurs différentes des éléments de fixation (3) alors que le diamètre du cylindre géométrique dans lequel s'inscrivent les sommets de filet reste constant.

## Patentansprüche

1. Vorrichtung zur gegenseitigen Befestigung von Knochenteilen, insbesondere Knochenteilen kleiner Größe, beispielsweise von Metatarsalkopfteilen, die die Form eines Dorns hat, der einen langgestreckten Schaft (1) aufweist, auf den nach einer Materialschwächungszone (2), die die Durchtrennung gestattet, ein Befestigungselement (3) folgt, das sich kegelstumpfförmig erstreckt, so dass der Durchmesser in Richtung auf das freie vordere Ende (4) des Elements zu abnimmt, und auf dieser kegelstumpfförmigen Fläche ein Gewinde aufweist, dessen Steigung von dem vorderen Ende an allmählich abnimmt und bei dem die Scheitel der Gewindegänge, deren Böden von dieser kegelstumpfförmigen Fläche gebildet werden, einem zur Achse des Dorns koaxialen geometrischen Zylinder (D) eingeschrieben sind, wobei das vordere Ende (4) so abgeschrägt ist, dass ein schneidendes Ende gebildet wird, um ein Einschrauben in die Knochenteile zu gestatten,
**dadurch gekennzeichnet, dass** der hintere Teil des Befestigungselements (3), der sich auf die Schwächungszone (2) zu erstreckt, Längsnuten (5) aufweist, die die Gewindegänge in dieser Zone teilweise unterbrechen, um die Einführung von entsprechenden Fingern eines Werkzeugs zu gestatten, das die Ausschraubung des Befestigungselements durch Drehung in einer zur selbstschneidenden Drehung umgekehrten Richtung gestattet.

2. Vorrichtung nach Anspruch 1, **dadurch gekennzeichnet, dass** der durchtrennbare Teil in Form einer Nut (2) mit im wesentlichen dreieckigem Querschnitt (4) ausgeführt ist, dessen Spitze einen im wesentlichen spitzen Nutboden bildet, der das Brechen des Befestigungselements durch Winkelverschwenkung bezüglich des Schafts gestattet, wobei gleichzeitig eine hohe Festigkeit gegenüber den bei der Eintreibung des Befestigungselements in die Knochenteile erzeugten Belastungen gewährleistet wird.

3. Vorrichtung nach einem der Ansprüche 1 bis 2, **dadurch gekennzeichnet, dass** die Querschnitte der Gewindegänge im wesentlichen dreieckig sind und eine geneigte Seite auf das vordere Ende (4) des Befestigungselements zu und eine zur Achse des Dorns im wesentlichen senkrechte Seite auf den hinteren Teil des Befestigungselements (3) zu aufweisen.

4. Satz von Vorrichtungen nach einem der Ansprüche 1 bis 3, **dadurch gekennzeichnet, dass** er eine Vielzahl von Vorrichtungen aufweist, bei denen die Abmessungen des Befestigungselements (3) in mindestens einem der folgenden Parameter variieren: Länge, Durchmesser der Gewindegangscheitel, Konizität, Steigung.

5. Satz nach Anspruch 4, **dadurch gekennzeichnet, dass** die Vorrichtungen des Satzes verschiedene Längen der Befestigungselemente (3) aufweisen, während der Durchmesser des geometrischen Zylinders, dem die Gewindegangscheitel eingeschrieben sind, konstant bleibt.

## Claims

1. Device for holding bone parts in position relative to one another, in particular small bone parts, for example, metatarsal head parts, which is configured in the form of a pin having an elongated stem (1) and, after a zone where the material narrows (2) to allow it to be severed, there follows a fixation element (3) extending in a tapering fashion to decrease in diameter in the direction of the free front end (4) of the element, and having on this tapering surface a thread, the pitch of which decreases progressively from said front end and wherein the crests of the threads, the roots of which are formed by said tapering surface, are inscribed in a geometric cylinder (D) that is coaxial to the axis of the pin, said front end (4) being bevelled in order to form a piercing end to enable it to be screwed into the bone parts, **characterised in that** the rear part of the fixation element (3), which extends towards the narrowed zone (2), has longitudinal grooves (5), which partially interrupt the threads in this zone to allow the introduction of corresponding prongs of a tool, which allows the fixation element to be unscrewed by rotating it in contrary direction to the self-cutting rotation.

2. Device according to Claim 1, **characterised in that** the part to be severed is configured in the form of a throat (2) of essentially triangular cross-section (4), the point of which determines an essentially acute throat base, which allows it to be broken by tilting the fixation element at an angle relative to the pin, while assuring perfect resistance to the stresses generated when the fixation element is driven into the bone parts.

3. Device according to one of Claims 1 to 2, **characterised in that** the thread cross-sections are essentially triangular with a face inclined towards the front end (4) of the fixation element and a face essentially perpendicular to the axis of the pin towards the rear part of the fixation element (3).

4. Set of devices according to one of Claims 1 to 3, **characterised in that** it comprises a plurality of devices, wherein the dimensions of the fixation element (3) vary according to at least one of the parameters: length, diameter of the thread crest, conicity, pitch.

5. Set according to Claim 4, **characterised in that** the devices of the set have fixation elements (3) with different lengths, while the diameter of the geometric cylinder, in which the thread crests are inscribed, remains constant.
